# EUROPEAN PATENT APPLICATION

(11) **EP 4 023 157 A1**
(43) Date of publication of application: **06.07.2022**
(21) Application number: 20217989.1
(22) Date of filing: 31.12.2020
(51) Int. Cl.: A61B 5/113, A61B 5/00, A61B 5/087

(54) **GENERATING A MODEL OF THE AIRWAY OF A SLEEPING SUBJECT**

(71) Applicant: Koninklijke Philips N.V., 5656 AG Eindhoven (NL)
(72) Inventor: KAHLERT, Joachim Johannes, 5656 AE Eindhoven (NL); DOS SANTOS DA FONSECA, Pedro Miguel Ferreira, 5656 AE Eindhoven (NL); WILLARD, Nicolaas Petrus, 5656 AE Eindhoven (NL); GRASSI, Angela, 5656 AE Eindhoven (NL)
(74) Representative: Philips Intellectual Property & Standards

(57) **Abstract**

A system and method for generating a model of the airway of a sleeping subject is provided. The system comprises an interface adapted to obtain an airway model configured to represent respiratory airflow in the airway of the subject; a movement sensor arrangement configured to detect movement of the chest or abdomen of a subject; an airflow sensor arrangement configured to detect an airflow in the airway of the subject; and a processor arrangement. The processor arrangement is configured to determine a movement pattern from detected movement of the chest or abdomen of the subject; determine an airflow pattern from detected airflow in the airway of the subject and modify the airway model based on the movement pattern and the airflow pattern so as to generate a subject-specific airway model for the subject representing respiratory airflow in the airway of the subject in a sleep state. The system may be used for patient stratification and sleep apnea therapy selection, wherein sleep apnea therapy selection covers the primary selection of a single or combination therapy, as well as the dynamic adaption of the selected therapies during sleep.

## Description

### FIELD OF THE INVENTION

This present invention relates to the field of medical modelling, and more particularly to generating a model of the airway of a sleeping subject for the treatment of sleep apnea.

### BACKGROUND OF THE INVENTION

Sleep Apnea is an involuntary cessation of breathing that occurs when the subject (e.g. patient) is asleep. The disorder is multifactorial, wherein age, gender, body weight, cardiac insufficiencies, pulmonary diseases, seasonal infections, and life style contribute to, and worsen, the disorder.

Obstructive sleep apnea (OSA) is characterized by a narrowing and collapse of the upper airway, wherein anatomical properties and features contribute mainly to the collapse. Beside static anatomical features, dynamic alterations of the upper airway also impede the narrowing of the airway. In particular, the gravitational displacement and swelling of soft tissue in the airway (palate, tongue, epiglottis) narrows the airway and thus increase the apnea-hypopnea index (AHI), a metric used to determine the severity of a subject's sleep apnea.

Central sleep apnea (CSA) is characterized by a decrease of the respiration drive without a change of the anatomical features of the upper airway and/or without a change of the airway flow resistance. The decrease of neural activity leads to a decline in tidal volume, a full cessation of airflow or a periodic breathing (Cheyne-Stokes Breathing).

The symptoms in both OSA and CSA are the change and modulation of tidal volume and minute volume (liters of breath per minute). The airflow reduction can be measured surrogately based on the blood oxygen desaturation, which causes a sympathetic response that is responsible for hemodynamic changes such as heart rate (HR), heart rate variability (HRV) and blood pressure (BP).

Breathing patterns in humans are generated from a combination of abdominal breathing (forced by the diaphragm) and chest breathing (forced by intrapleural muscles). In healthy persons without any breathing restrictions, the chest and abdominal movement is typically aligned in time. An opposite phase of abdominal and chest movement is typically indicative of an occluded airway and is used in a polysomnogram (PSG) to discriminate OSA from CSA. However, in hypopnea and/or a mixed form of OSA and CSA, an accurate diagnosis is currently impossible. There is not yet a specific correlation defined between a change in the breathing pattern and a certain physiological, neural or anatomical change. A quantified analysis and a definition of cutoff values for the therapy selection and device iteration is missing.

If the oral and/or nasal airflow is reduced during sleep, there is currently no way to determine the cause of the flow reduction. It may be caused by either a reduction of the respiration drive, by an increase of the airway resistance, by a partial narrowing of the upper airway in inspiration, by the gravitational impact of the abdominal fat and/or by the displacement of anatomical structures. Younger persons are typically able to compensate an increased airway resistance (e.g. when snoring) by generating increased respiration drive, whereas elderly persons are typically unable generate an adequate increase in respiration drive to compensate the increased airway resistance.

A primary aim of therapy for OSA and CSA is to eliminate the physiological stress that is caused by the airflow reduction. Such therapy is therefore typically adapted to try to rebalance the airflow in order to achieve a stable tidal volume and a constant minute volume during sleep.

Currently preferred therapy for OSA is a positive airway pressure therapy (CPAP) and the currently preferred therapy for CSA is an adaptive support ventilation by an Average Volume Assure Pressure Support (AVAPS) device. These therapies are normally effective for rebalancing the airflow. However, the continuous positive pressure in these OSA and CSA therapies can cause a further physiological stress.

### SUMMARY OF THE INVENTION

The invention is defined by the claims.

According to examples in accordance with an aspect of the invention, there is provided a system for generating a model of the airway of a sleeping subject, the system comprising:
an interface adapted to obtain an airway model configured to represent respiratory airflow in the airway of a subject;
a movement sensor arrangement configured to detect movement of the chest or abdomen of a subject;
an airflow sensor arrangement configured to detect an airflow in the airway of the subject; and
a processor arrangement configured to:
   determine a movement pattern from detected movement of the chest or abdomen of the subject;
   determine an airflow pattern from detected airflow in the airway of the subject;
   modify the airway model based on the movement pattern and the airflow pattern so as to generate a subject-specific airway model for the subject representing respiratory airflow in the airway of the subject in a sleep state.

Proposed embodiments may enable the generation of a model of the airway of a sleeping subject (e.g. a patient) using airflow measurements and chest/abdominal movement measurements, which are recorded using airflow/movement sensor arrangements. For instance, a processor can be employed to determine the airflow and movement patterns and generate a subject-specific airway model for the subject representing respiratory airflow in the airway of the subject in a sleep state. The model may be used to identify the anatomical structure and physiological changes that contribute to the OSA event. Furthermore, the model may be used to select an appropriate OSA therapy and to validate the efficacy of the chosen therapy. The model may also enable the dynamic adaption of the therapy device settings (and/or selected therapies) in order to provide optimal treatment, wherein OSA therapy solutions comprise positional therapy, positive airway pressure, oral applications and surgical interventions for example. An advanced analysis of the breathing pattern, chest/abdominal movements and the oral/nasal airflow is needed for a better patient stratification, therapy selection and dynamic titration of the therapy device settings.

In particular, it is proposed that a model of the airway of a subject may be obtained. Such a model may be generic, in that it is not specifically adapted to an individual subject and/or not specifically adapted to particular state or property of a subject. Embodiments proposed to adapt (i.e. modify, alter or otherwise change) the model of the airway so that it models the airway of a specific subject in a sleep state (i.e. a sleeping subject). By generating a subject-specific airway model for the subject representing respiratory airflow in the airway of the sleeping subject, embodiments may provide for more accurate sleep monitoring and/or improved sleep therapy for the subject.

In particular, embodiments may be used in relation to a specific subject so as to assist and/or optimize medical assessment, therapy and/or treatment for that subject. Such embodiments may support improved clinical assessment and/or planning. Improved Clinical Decision Support (CDS) may therefore be provided by proposed concepts.

A proposed embodiment may comprise a system or method that is configured correlate changes of breathing pattern and chest/abdominal movements to alterations of anatomical features, neural activity, sleep stage and body position. Since breathing pattern and chest/abdominal movements are person-specific, these patterns may be used for OSA stratification and OSA therapy device settings by generating a subject-specific airway model. For instance, the model may use patterns recorded in real-time during sleep and patterns measured as reference data. This reference data may, for example, be acquired during predefined breathing manoeuvers. Using the model, patient and disease stratification in sleep-disordered breathing (SDB), e.g. snoring/OSA/CSA, may be undertaken for therapy decision support and real-time adaptation of the therapy device settings and/or dynamic adaption of the selected therapies during sleep.

Proposed embodiments may use subject-specific measurements to determine the most appropriate treatment for the subject. For example, increased respiration drive, which is characterized by an increased sympathetic activity, may be measured. This may impede the sleep stage and lower the sleep quality. In this case, there is no apnea/hypopnea event and there is no need for OSA treatment. However, an OSA therapy like positional therapy counteracts a gravitational displacement of anatomical structure. The needed respiration drive is lower and the person has to compensate less to balance the airflow. Consequently, the sympathetic activity declines and the sleep quality improves.

Embodiments may disclose methods of improving the sleep quality in persons who are able to compensate and do not suffer from blood oxygen desaturation. Other embodiments may determine a threshold level, at which the person is no longer able to compensate and a further sleep apnea therapy is reasonable.

If an apnea event or a desaturation of the blood oxygen level SpO2 is detected, there is a need for a therapy. However, in a multifactorial disorder it is not obvious which therapy is the best and which parameter are the most optimal device settings. The movement of the chest and abdomen and the oral/nasal airflow give specific information as to the cause of the airflow reduction. Therefore, as previously mentioned, to provide optimal OSA therapy, a subject-specific airway model must be generated.

An embodiment may comprise a positional therapy device as a primary platform for obtaining detecting movement of the subject. The embodiment may further comprise a second belt around the abdomen and a nasal/oral flow sensor.

Proposed embodiments may be used to enhance a sleep study, for example a polysomnogram (PSG) or a home sleep test (HST).

Some embodiments may comprise: a chest belt, an abdominal belt, an oral flow sensor, two nasal flow sensors, an accelerometer, a computer model and equivalent circuit and a processing unit. The accelerometer may be used to sense dynamic movements and the static position of the chest, neck and head. The computer model and equivalent circuits may be used to describe the inspiration and expiration in a sleeping subject (i.e. a human subject in a sleep state). Such embodiments may also be extended using an interface to a PSG resp. HST system, a photoplethysmogram (PPG) sensor, an electrocardiogram (ECG) sensor, an electroencephalography (EEG) sensor, an electromyography (EMG) sensor, a microphone or an OSA therapy device (CPAP - continuous positive airway pressure, MAD - mandibular advancement devices, PT - positional therapy).

Embodiments may be used to detect, monitor and quantify alterations during sleep, wherein these alterations impede the oral/nasal airflow and the chest/abdominal movement.

Proposed embodiments may enable the use/leverage of an increased quantity of information (e.g. movement of chest and abdomen and oral/nasal airflow) regarding a subject's breathing pattern. Using this information, improved diagnosis may be undertaken to determine the cause of the airflow reduction. Additionally, or alternatively, a threshold level may be determined that for identifying whether a subject requires treatment or not. In the case of a subject requiring treatment, the increased quantity of information may be utilized to make more informed treatment decisions. As a result, subjects may benefit from more appropriate therapy selection (which may be more effective than the administration of conventional therapies).

Further, by recording the breathing patterns and responses to treatment of a plurality of subjects, a library of patterns/responses may be created in order to improve treatment for future subjects with similar breathing patterns. Hence, the efficacy of treatments for future subjects can be improved. Additionally, by recording the response of subjects to certain treatments, more appropriate treatments may be selected for the subjects in the future. For example, if a subject responds poorly to physical therapy, other therapies relating to physical therapy may not be recommended to the subject again.

In some embodiments, the airway model may comprise a circuit arrangement configured to represent the airway of a subject. The circuit arrangement may comprise one or more passive circuit elements and one or more active circuit elements. Each passive circuit element may be configured to represent a respective anatomical structure of the airway. Each active circuit element may be configured to represent a respective driving force for airflow in the airway. By assigning circuit elements to structures and driving forces throughout the subject's airway, a detailed and consistent subject-specific airway model may be generated. Further, such a circuit arrangement may be simple to adapt or change in order to reflect physiological changes that are specific to the subject.

For example, in some embodiments, modifying the airway model may comprise determining a value of airway tissue compliance based on at least one of the movement pattern and the airflow pattern; and modifying at least one of the one or more passive circuit elements based on the determined value of airway tissue compliance. Hence, the passive circuit elements may be modified as the structure of the subject's airway changes, such that the subject-specific airway model is kept up-to-date.

In some embodiments, modifying the airway model may comprise determining a value of respiration drive based on at least one of the movement pattern and the airflow pattern; and modifying at least one of the one or more active circuit elements based on the determined value of respiration drive. In this way, the active circuit elements may be modified as the subject's respiration drive changes, such that the subject-specific airway model is kept up-to-date.

Also, in some embodiments, modifying the airway model may comprise determining a value of airflow based on at least one of the movement pattern and the airflow pattern; and modifying at least one of the one or more passive circuit elements based on the determined value of airflow. The passive circuit elements may therefore be modified as the subject's airflow varies, such that the subject-specific airway model is kept up-to-date.

In some embodiments, one or more passive circuit elements may comprise a complex impedance for representing an airway segment of the airway. The real part of the complex impedance may be configured to represent a dimension of the airway segment. The imaginary part of the complex impedance may be configured to represent an airflow compliance of the airway segment. Hence, the passive circuit elements may provide a more detailed representation of the airway segment.

In other embodiments, one or more active circuit elements may comprise a current source for representing a muscular-based respiration drive for airflow in the airway.

In further embodiments, one or more active circuit elements may comprise at least one of: a voltage source for representing a fixed respiration drive for airflow in the airway; a voltage source for representing a positive airway pressure therapy for airflow in the airway; and a voltage source for representing a mechanical ventilation therapy for airflow in the airway. Various modes of therapy may therefore be modelled in the subject-specific airway model, and the most appropriate therapy for the subject may then selected accordingly.

Other embodiments may comprise a supplementary sensor arrangement configured to detect a physiological characteristic of the subject. The processor arrangement may be further adapted to generate the subject-specific airway model for the subject further based on the detected physiological characteristic of the subject. This may facilitate the generation of a more accurate subject-specific airway model of the subject.

In further embodiments, the supplementary sensor arrangement may comprise at least one of: an ECG sensor for detecting volume overload; a heart rate sensor; an oxygen saturation sensor; a microphone; a PPG sensor; an EEG sensors; and an EMG sensor. A wide variety of sensors may therefore be employed by embodiment, thus facilitating the leverage of many different types or forms of data for improving model generation.

In some embodiments, the processor may be further adapted to analyses, with the subject-specific airway model, at least one of either a detected movement of the chest or abdomen of the subject, or a detected airflow in the airway of the subject. An analysis result may then be obtained and an output signal based on the analysis result may be generated.

In further embodiments, the output signal may comprise at least one of: an alert for instructing a user or system to undertake an action; a control instruction for a therapy system; or one or more configuration settings for a system. Hence, the subject may be updated in real time with the progress of their therapy system.

Some embodiments may comprise a sleep therapy system, which comprises a system for generating a model of the airway of a subject according to any preceding claim; and a controller configured to analyses a subject-specific airway model and to generate a control signal based on the analysis result.

In other embodiments, the sleep therapy system may further comprise a breathing ventilation system, wherein the controller is further adapted to control the breathing ventilation system based on the analysis result.

Embodiments may comprise a method for generating a model of the airway of a sleeping subject. The method may comprise: obtaining an airway model configured to represent respiratory airflow in the airway of a subject; detecting movement of the chest or abdomen of a subject; detecting an airflow in the airway of the subject; determining a movement pattern from detected movement of the chest or abdomen of the subject; determining an airflow pattern from detected airflow in the airway of the subject; modifying the airway model based on the movement pattern and the airflow pattern so as to generate a subject-specific airway model for the subject representing respiratory airflow in the airway of the subject in a sleep state.

Embodiments may also comprise a method for controlling a sleep therapy system, the method comprising: generating a model of the airway of the subject; analyzing the subject-specific airway model; and generating a control signal based on the analysis result.

Some embodiments may comprise a computer program comprising computer program code which is adapted, when said program is run on a computer, to implement either the method for generating a model of the airway of the sleeping subject, or the method for controlling a sleep therapy system.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows an equivalent circuit of upper airway according to a proposed embodiment;
Figure 2 shows an equivalent circuit of lower airway according to a proposed embodiment;
Figure 3 shows a system for generating a model of the airway of a sleeping subject according to a proposed embodiment;
Figure 4 shows a representation of an airway model according to an embodiment;
Figure 5 is a flow diagram of method for generating a model of the airway of a sleeping subject according to an embodiment;
Figure 6 shows a flow diagram of a method for controlling a sleep therapy system according to an embodiment; and
Figure 7 illustrates an example of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Proposed embodiments may enable the generation of a model of the airway of a sleeping subject (e.g. a patient) using airflow measurements and chest/abdominal movement measurements, which are recorded using airflow/movement sensor arrangements. Hence, a processor can determine the airflow and movement patterns and generate a subject-specific airway model for the subject representing respiratory airflow in the airway of the subject in a sleep state. The model may, for example, be used to identify one or more anatomical structure and physiological changes that contribute to an OSA event. Furthermore, the model may be used to select an appropriate OSA therapy and to validate the efficacy of the chosen therapy. Also, a model generated according to a proposed embodiment may enable the dynamic adaption of OSA therapy settings in order to provide optimal treatment, wherein OSA therapy solutions comprise positional therapy, positive airway pressure, oral applications and surgical interventions. Embodiments may therefore provide for improved patient stratification, therapy selection and dynamic titration of the therapy device settings.

According to proposed embodiments, the movement of the lungs can be modelled by a breathing model, used to predict inspiration and expiration. Such a model comprises a ribcage, a sternum, costal cartilages connecting the ribs to the sternum, intercostal muscles between ribs, and a diaphragm.

During active inspiration, the external intercostal muscles and diaphragm both contract. This contraction causes the expansion of the ribs as the chest wall and lungs expand. During active expiration, the external intercostal muscles relax, the internal intercostal muscles and abdominals contract and the diaphragm relaxes. This relaxation causes the depression of the ribs as the chest cavity and lungs contract. For active inspiration/expiration, chest breathing is forced by the intercostal muscle, and the driving force is modelled by a voltage representing the intrapleural pressure. Abdominal breathing is forced by the diaphragm, and the driving force is modelled by a voltage source representing the movement of the diaphragm.

During passive expiration, the external intercostal muscles and diaphragm relax, though notably the internal intercostal muscles and abdominals do not contract. The recoiling of the chest is modelled by a voltage source, and the diaphragm movement representing the abdominal movement is modelled by a voltage source representing the gravitational forces driven by the mass of the belly.

If ventilation support is required, pressure therapy provided by a CPAP (Continuous Positive Airway Pressure) device is modelled by a voltage source representing the positive pressure of the device. The volume support therapy provided by a mechanical ventilator is modelled by a current source representing the airflow drive by a mechanical ventilator.

The human airway is split into the upper airway and the lower airway. The upper airway, also known as the extrathoracic airway, is from the nostrils/lips to the epiglottis. The lower airway, also known as the thoracic or pulmonary airway, is from the epiglottis to the alveoli.

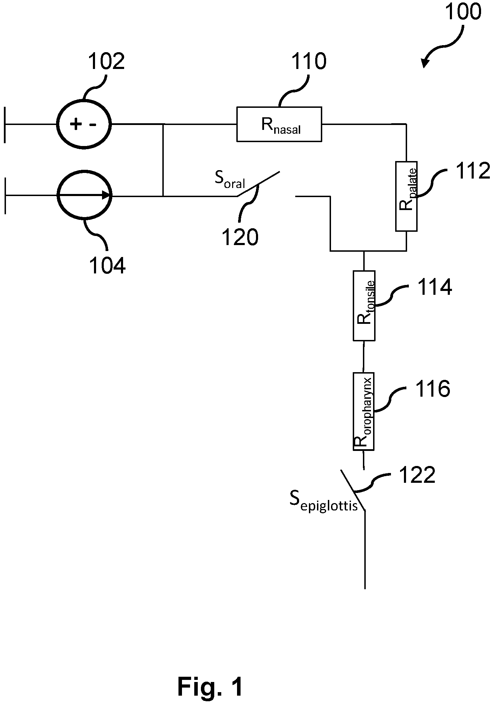

The upper airway model may be represented by the upper airway equivalent circuit 100 illustrated in Figure 1. The nasal airway is modelled by a resistive element Rₙₐₛₐₗ 110, wherein a clotted nose is modelled by an increase in Rₙₐₛₐₗ. The time constant for a seasonal infection is in the order of days, whereas the time constant for a nasal cycle is in the order of hours. The resistance of the oral airway is much lower than that of the nasal airway, and hence is represented by a switch Sₒᵣₐₗ 120, such that when the switch is closed, the resistance is 0, i.e. Rₒᵣₐₗ = 0. The palatal area is modelled as a resistive element Rₚₐₗₐₜₑ 112. The collapse at the palatal area happens within one breathcycle, hence the resistance toggles between Rₚₐₗₐₜₑ and infinite. The tonsils are modelled as a resistive element Rₜₒₙₛᵢₗₑ 114. The oropharyngeal area is also modelled as a resistive element R_{oropharynx} 116, and the resistive value increase from baseline to infinite (in case of a collapse), since the oropharyngeal airway is narrowed by the posterior displacement of the tongue base. The epiglottis is modelled as a switch S_{epiglottis} 122 that can move between open and closed.

Additionally, in this example, the equivalent circuit has a CPAP device that is modelled by a voltage source 102 representing the positive pressure of the device. The equivalent circuit also has a mechanical ventilator that is modelled by a current source 104 representing the airflow drive by a mechanical ventilator.

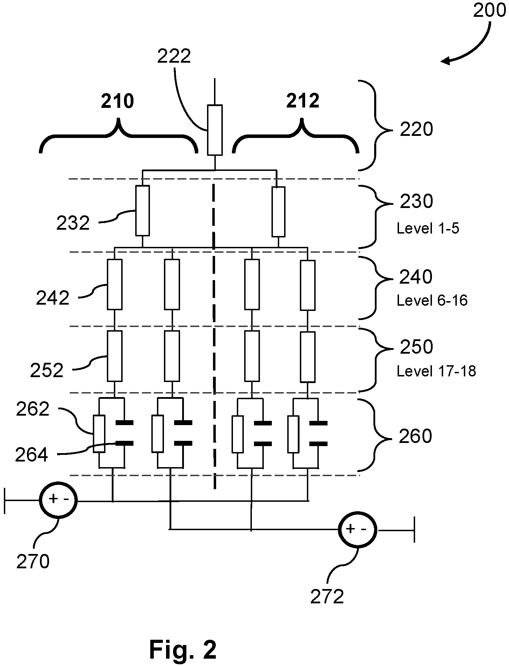

The lower or pulmonary airway 200 may be represented by the lower airway equivalent circuit is illustrated in Figure 2. As depicted, the lower airway equivalent circuit 200 and comprises the following structures: the lungs 210, 212, the trachea 220, the bronchi 230, 240, and the bronchial tree comprising bronchioli 250 and alveoli 260. The left lung 210 and right lung 212 comprise identical sets of resistive/capacitive elements.

The trachea 220 and larger bronchi 230 (i.e. bronchi (level 1-5)) are conductive, stiff elements which do not collapse, and hence are represented by resistive elements R_{trachea} 222, R_{bronchi(1-5)} 232. Whilst the trachea 220 is not part of the lung, its similar anatomical structure means it can be modelled like the larger bronchi 230.

The larger bronchi 230 (level 1-5) are conductive airways with low compliance that do not collapse. Hence, these bronchi 230 are represented by static resistive elements, such that R_{bronchi(1-5)} 232 = R_{bronchi(large)}.

The medium bronchi 240 (level 6-16) are conductive and do not collapse. However, they can narrow due to accumulation of mucus. These bronchi 240 are represented by a quasistatic resistive element R_{bronchi(6-16)} 242 = R_{bronchi(medium)}. The time constant for dynamic change is in the order of days.

The bronchioli 250 (level 17-18) are resistive and can narrow quickly (i.e. in minutes), for instance in the case of asthma or mucus. In COPD (chronic obstructive pulmonary disease) patients, these bronchioli can collapse. These bronchioli 250 are represented by a resistive element R_{bronchioli(17-18)} 252 = R_{bronchioli(small)}. A collapse is modelled by equating resistance to 0, i.e. R_{bronchioli(small)} = 0.

The alveoli 260 are compliant structures, hence the resistive component Rₐₗᵥₑₒₗᵢ 262 is low. The capacitor Cₐₗᵥₑₒₗᵢ 264 represents the stiffness of the anatomical structure. The Cₐₗᵥₑₒₗᵢ 264 value depends on the comorbidities of COPD and edema (fluid accumulation).

Abdominal breathing is forced by the diaphragm muscles, and the driving force is modelled by a voltage source 270 representing the movement of the diaphragm. Chest breathing is forced by intercostal muscle, and the driving force is also modelled by a voltage source 272 representing the intrapleural pressure.

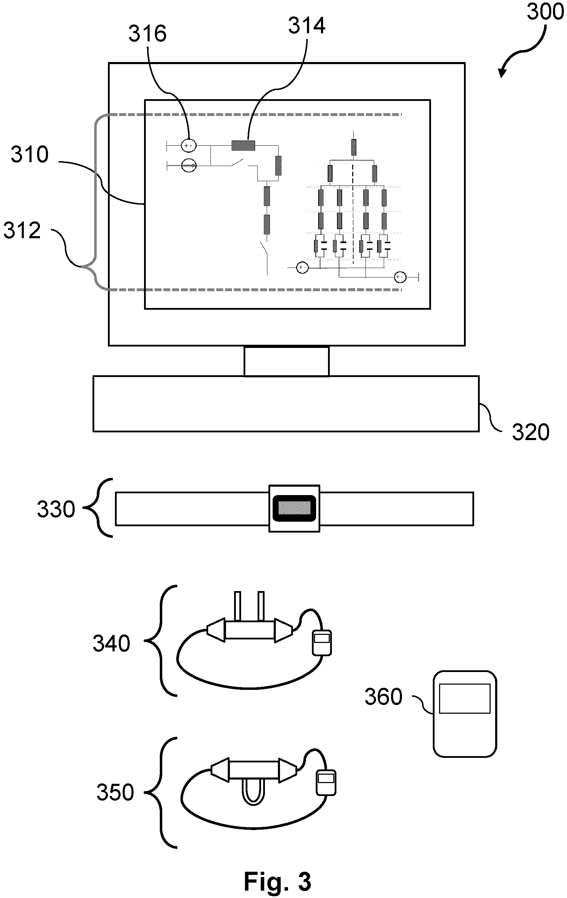

Figure 3 is a simplified block diagram of a system 300 for generating a model 312 of the airway of a sleeping subject according to an embodiment.

The system 300 comprises an interface 310, a movement sensor arrangement 330, an airflow sensor arrangement 340, a supplementary sensor arrangement 350, a processor arrangement 320 and a controller 360.

The interface 310 is adapted to obtain an airway model 312 configured to represent respiratory airflow in the airway of a subject. The movement sensor arrangement 330 is configured to detect movement of the chest or abdomen of a subject. The airflow sensor arrangement 340 is configured to detect an airflow in the airway of a subject.

The processor arrangement 320 is configured to determine a movement pattern from movement of the chest or abdomen of the subject detected by the movement sensor arrangement 330. The processor arrangement 320 is further configured to determine an airflow pattern from airflow in the airway of the subject detected by the airflow sensor arrangement 340.

The processor arrangement 320 is configured to modify the airway model 312 based on the movement pattern and the airflow pattern so as to generate a subject-specific airway model 312 for the subject representing respiratory airflow in the airway of the subject in a sleep state. Specifically, the airway model 312 comprises a circuit arrangement configured to represent the airway of the subject, wherein the circuit arrangement comprises one or more passive circuit elements 314 and one or more active circuit elements 316. Each passive circuit element is configured to represent a respective anatomical structure of the airway. Each active circuit element is configured to represent a respective driving force for airflow in the airway.

The group of one or more passive circuit elements 314 comprises a complex impedance for representing an airway segment of the airway. The real part of the complex impedance is configured to represent a dimension of the airway segment. The imaginary part of the complex impedance is configured to represent an airflow compliance of the airway segment.

The group of one or more active circuit elements 316 comprises a current source for representing a muscular-based respiration drive for airflow in the airway. The group of one or more active circuit elements 316 further comprises at least one voltage source, wherein the voltage source has different representations. Here, voltage source represents either: a fixed respiration drive for airflow in the airway; a positive airway pressure therapy for airflow in the airway; or a mechanical ventilation therapy for airflow in the airway.

Modifying the airway model 312 comprises determining a value of airway tissue compliance based on at least one of the movement pattern and the airflow pattern and then modifying at least one of the one or more passive circuit elements 314 based on the determined value of airway tissue compliance. Modifying the airway model 312 also comprises determining a value of respiration drive based on at least one of the movement pattern and the airflow pattern and then modifying at least one of the one or more active circuit elements 316 based on the determined value of respiration drive. Modifying the airway model 312 even further comprises determining a value of airflow based on at least one of the movement pattern and the airflow pattern and then modifying at least one of the one or more passive circuit elements 314 based on the determined value of airflow.

The system 300 of this embodiment also comprises a supplementary sensor arrangement 350 configured to detect a physiological characteristic of the subject.The processor arrangement 320 is further adapted to generate the subject-specific airway model 312 for the subject based on the detected physiological characteristic of the subject. By way of example, the supplementary sensor arrangement may comprise at least one of: an ECG sensor for detecting volume overload; a heart rate sensor; an oxygen saturation sensor; a microphone; a PPG sensor; an EEG sensor; and an EMG sensor.

The processor arrangement 320 can be further adapted to analyses, with the subject-specific airway model 312, at least one of the detected movement of the chest or abdomen of the subject and/or detected airflow in the airway of the subject, so as to obtain an analysis result. An output signal is then generated based on the analysis result. The output signal comprises at least one of: an alert for instructing a user of system to undertake an action; a control instruction for a therapy system; and/or one or more configuration settings for a system.

The system 300 for generating a model of the airway of a subject may also be used in conjunction with a controller 360. The controller 360 is configured to analyses a subject-specific airway model 312 and to then generate a control signal based on the analysis result. The combination of the system 300 and the controller 360 may therefore provide a sleep therapy system. Such sleep therapy system may, for example, further comprise a breathing ventilation system, and the controller 360 may be adapted to control the breathing ventilation system based on the analysis result.

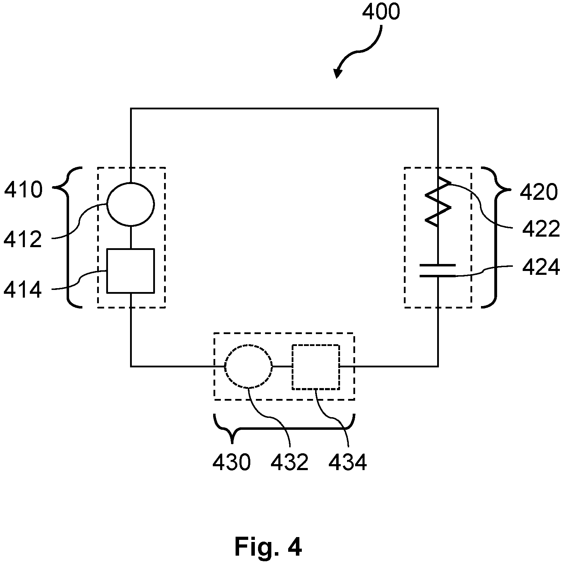

Figure 4 shows a representation 400 of an airway model according to an embodiment (e.g. the airway model 312 from Figure 3). The airway model comprises active circuit elements 410, o passive circuit elements 420 and circuit extensions 430.

Each active element 410 represents a respective driving force for inflow and outflow of air. In the example of Figure 4, the active circuit elements 410 comprises a current source 412 for representing a muscular-based respiration drive for airflow in the airway. A current source can describe the muscular activity, for example the respiration drive for inspiration and expiration, or can represent the active neural control of inspiration. The active circuit elements 410 further comprises one or more voltage sources 414, wherein the voltage sources have different representations. For instance, a voltage source may represent a fixed respiration drive for airflow in the airway. A voltage source may also represent a positive airway pressure therapy for airflow in the airway. Another voltage source may represent a mechanical ventilation therapy for airflow in the airway. That is, a voltage source can be used to represent a static or quasi-static force which acts on the compliant airway compartments i.e. the voltage source forces the rushing out of air. One example is the passive recoiling of the chest. A further example is the gravitational force of the bulk of the abdominal fat, which elevates the diaphragm.

Each passive element 420 of the equivalent circuit represents a respective anatomical structure of the upper and lower airway. In the example of Figure 4, the passive circuit elements 420 comprise a complex impedance for representing an airway segment of the airway. The complex impedance represents the airflow resistance, wherein the complex impedance comprises a real part 422 and an imaginary part 424. The real part 422 of the complex impedance is configured to represent a dimension of the airway segment. The real part may also represent the increase/decrease of airflow resistor. Further, the real part 422, which determines the resistive behavior, may be given by the cross-section and length of each airway segment. The imaginary part 424 of the complex impedance is configured to represent an airflow compliance of the airway segment. Further, the imaginary part 424, which determines the capacitive behavior, may be given by the tissue compliance and air volume in a compliant airway segment.

The equivalent circuit further comprises extensions to the circuit 430, for example, to model ventilation support devices. The extensions to the circuit comprise additional current sources 432 that represent mechanical ventilation therapy. The extensions to the circuit also comprise additional voltage sources 434 that represent positive airway pressure therapies (CPAP, APAP BiPAP).

Based on obtained movement patterns and airflow patterns for a monitored subject, the elements of the airway model of Figure 4 can be modified so as to generate a subject-specific airway model 550 for the subject representing respiratory airflow in the airway of the subject in a sleep state. In this way, a generic airway model may be tailored to the unique properties, characteristics and/or circumstances of a subject.

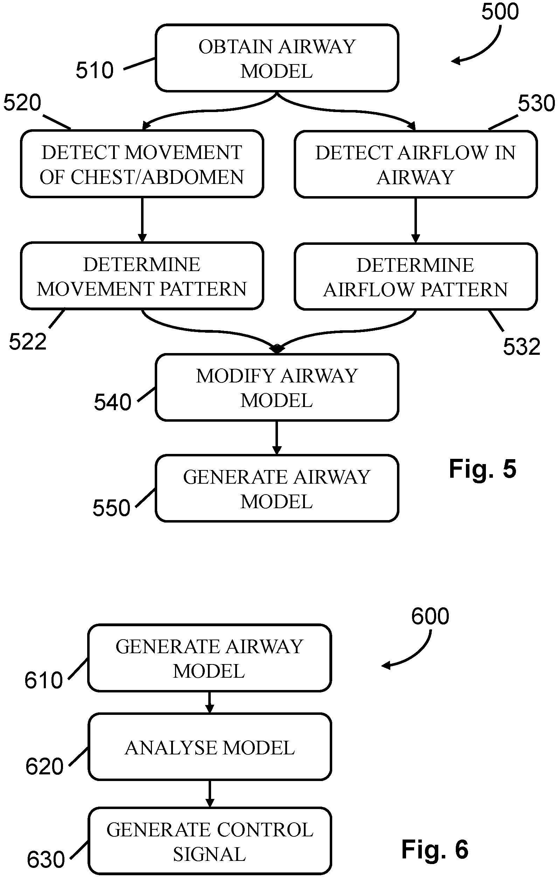

Referring now to Figure 5, an exemplary method for generating such a subject-specific airway model according to an embodiment will now be described.

Figure 5 shows a flow diagram of method 500 for generating a model of the airway of a sleeping subject according to an embodiment. The method 500 comprises obtaining 510 an airway model, wherein the airway model is configured to represent respiratory airflow in the airway of a subject. The method also comprises detecting 520 movement of the chest or abdomen of the subject and detecting 530 airflow in the airway of the subject. In step 522, a movement pattern determined from the detected movement of the chest or abdomen of the subject. Similarly, in step 532, an airflow pattern is determined from the detected airflow in the airway of the subject 530. The airway model is then modified 540 based on the determined movement pattern and the determined airflow pattern 532 so as to generate a subject-specific airway model 550 for the subject representing respiratory airflow in the airway of the subject in a sleep state. Examples of how an airway model may be modified according to determined movement pattern and/or airflow flow patterns are detailed below.

The alterations of airflow and chest/abdominal movement may be analyzed in the time domain and in the frequency domain. The change of the spectrum in the frequency domain and the change of the pulse in the time domain (rising/falling slope, amplitude, overshoot, dips, and duty cycle) are specific for a certain physiological change.

Embodiments may employ a system that monitors oral and nasal airflow. Other embodiments may employ a system that monitors chest and abdominal movements. These measurements may be complemented by data acquired by the movement and airflow sensors.

Physiological alterations may then be extracted from flow and movement changes, for example, expiration under rest is the passive outflow of air from the lung driven by the recoiling of the chest and driven by gravitational forces. Further, inspiration is always an active inflow of air into the lung driven by the diaphragm and the intrapleural muscles.

The passive outflow of air may be indicated by a change in resistance of the resistor representing the upper and lower airway, a change in compliance of palate, pharynx and bronchi/alveoli or a change of driving force e.g. body position related changes of gravitational changes.

The active inflow of air may be indicated by a change in resistance of the resistor representing the upper airway e.g. displacement of tongue, plate in OSA, swelling/clotting of nasal airway. The active inflow of air may also be indicated by a change of compliance, e.g. a change of muscle tone in upper airway, or a change of chest in respect to abdominal breathing.

By way of example, an embodiment may be configured to represent passive expiration as an RC circuit with a switched voltage source, wherein the frequency is the respiration rate and the duty cycle (the pulse duration) is the length of the expiration cycle. The measured data may be analyzed with respect to a reference baseline measurement from an awake person. An increase in airway resistance, e.g. the narrowing of the airways, may limit airflow and cause a reduction in peak flow. Hence the data may be extracted in the time domain from the flow pattern (amplitude, rise time). A change in airway tissue compliance may cause a change in capacitive behavior. The data may also be extracted from the frequency domain (spectrum, low pass/high pass filtering of breathing pattern). A change in respiration drive may cause a chance in driving force (voltage source). The data may be analyzed in the time/frequency domain (amplitude attenuation without change of the frequency spectrum).

By way of further example, an embodiment may be configured to represent active inspiration as an RC circuit with a switched current source. Ventilation support by a CPAP device may be modelled as a further voltage source. An increase in airway resistance may be analyzed in the time domain by the flow limitation (amplitude, rise time). A decrease in respiration can be analyzed in the time/frequency domain, for example the respiration decline may lead to a decline in amplitude both in the time domain and frequency domain without a change of the normalized spectrum. A change in airway tissue compliance may cause a change in capacitive behavior. The data may be extracted from the frequency domain (spectrum, low pass/high pass filtering of breathing pattern). A change in respiration drive may cause a chance in driving force (voltage source). The data may also be analyzed in the time/frequency domain (amplitude attenuation without change of the frequency spectrum).

Some embodiments may be configured to represent assisted ventilation support (CPAP or mechanical ventilation) as partial air flow changes. The pressure of the CPAP device may have a larger decrease in steps from breath cycle to breath cycle. The incremental changes of flow pattern and chest/abdominal movements may be analyzed in the time and frequency domains. A computer unit may be used to post-process the acquired data for a fitting of the elements of the airway model.

An embodiment may be configured to distinguish between congestion and obstruction/airway collapse. For instance, a congestion may be characterized by a static or quasi-static narrowing of one or more segments of the upper airway. A typical congestion may be a seasonal infection that narrows one or both nostrils, wherein the narrowing is slow with respect to time. Hence, the breathing rate can be considered as being constant for some consecutive breath cycles. The narrowing during the nocturnal nasal cycle, i.e. the periodic narrowing of the left or right nostril is similar and has a typical periodical duration of one/two hours. The swelling of the tonsils may also be considered a static narrowing, wherein the narrowing can last for multiple days and is constant in the inspiration and expiration cycle. The narrowing is independent of the breathing cycle-related pressure modulation in the airway (i.e. the positive pressure during expiration and negative pressure during inspiration). An obstruction/airway collapse may be identified by a dynamic narrowing of the airway which is synchronized to the breathing cycles. Hence, the narrowing may depend on the intrinsic negative pressure during inspiration and the positive pressure during expiration. An analysis in the frequency domain may be used to distinguish between congestion and obstruction/airway collapse. The information can be extracted from the frequency spectrum of the airflow signal. The information may also be extracted in the time domain from the ratio of rise time in inspiration cycle and falling time in expiration.

In conservative OSA therapies (like existing positional therapy) an advanced solution requiring more than one decision criteria are reasonable. For example, the primary decision criteria for a positional change may be the supine body position, and the secondary decision criteria may depend on restricted breathing events. The new concept will be to trigger a positional change only in the case of both the primary and secondary criteria being given. This concept may also be used for CPAP OSA therapy.

Figure 6 shows a flow diagram of a method 600 for controlling a sleep therapy system. The method 600 comprises generating a model of the airway of the subject 610 (wherein step 610 corresponds to step 550 of Figure 5). The subject-specific airway model is then analyzed 620 and a control signal is generated 630 based on the analysis result. Thus, it will be understood that embodiments may support control and/or adaptation of the therapy systems or device settings. Such control and/or adaption may be undertaken dynamically and in real-time.

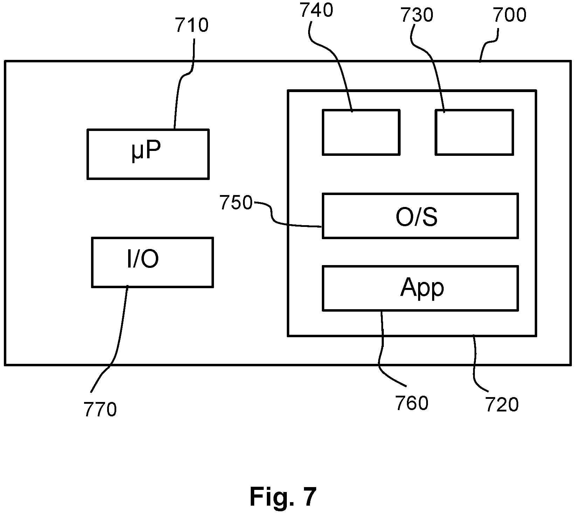

By way of further example, Figure 7 illustrates an example of a computer 700 within which one or more parts of an embodiment may be employed. The computer 700 may be personalized, for example, the baseline parameters of the model may be extracted from reference measurements in rest while the person is awake. Optionally, breathing manoeuvres may be performed according to a predefined protocol. Various operations discussed above may utilize the capabilities of the computer 700. For example, a system for generating a model of the airway of a sleeping subject may be incorporated in any element, module, application, and/or component discussed herein. In this regard, it is to be understood that system functional blocks can run on a single computer or may be distributed over several computers and locations (e.g. connected via internet).

The computer 700 includes, but is not limited to, PCs, workstations, laptops, PDAs, palm devices, servers, storages, and the like. Generally, in terms of hardware architecture, the computer 700 may include one or more processors 710, memory 720, and one or more I/O devices 770 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 710 is a hardware device for executing software that can be stored in the memory 720. The processor 710 can be virtually any custom made or commercially available processor, a central processing unit (CPU), a digital signal processor (DSP), or an auxiliary processor among several processors associated with the computer 700, and the processor 710 may be a semiconductor based microprocessor (in the form of a microchip) or a microprocessor.

The memory 720 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 720 may incorporate electronic, magnetic, optical, and/or other types of storage media. Note that the memory 720 can have a distributed architecture, where various components are situated remote from one another, but can be accessed by the processor 710.

The software in the memory 720 may include one or more separate programs, each of which comprises an ordered listing of executable instructions for implementing logical functions. The software in the memory 720 includes a suitable operating system (O/S) 750, compiler 740, source code 730, and one or more applications 760 in accordance with exemplary embodiments. As illustrated, the application 760 comprises numerous functional components for implementing the features and operations of the exemplary embodiments. The application 760 of the computer 700 may represent various applications, computational units, logic, functional units, processes, operations, virtual entities, and/or modules in accordance with exemplary embodiments, but the application 760 is not meant to be a limitation.

The operating system 750 controls the execution of other computer programs, and provides scheduling, input-output control, file and data management, memory management, and communication control and related services. It is contemplated by the inventors that the application 760 for implementing exemplary embodiments may be applicable on all commercially available operating systems.

Application 760 may be a source program, executable program (object code), script, or any other entity comprising a set of instructions to be performed. When a source program, then the program is usually translated via a compiler (such as the compiler 740), assembler, interpreter, or the like, which may or may not be included within the memory 720, so as to operate properly in connection with the O/S 750. Furthermore, the application 760 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, NET, and the like.

The I/O devices 770 may include input devices such as, for example but not limited to, a mouse, keyboard, scanner, microphone, camera, etc. Furthermore, the I/O devices 770 may also include output devices, for example but not limited to a printer, display, etc. Finally, the I/O devices 770 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 770 also include components for communicating over various networks, such as the Internet or intranet.

If the computer 700 is a PC, workstation, intelligent device or the like, the software in the memory 720 may further include a basic input output system (BIOS) (omitted for simplicity). The BIOS is a set of essential software routines that initialize and test hardware at startup, start the O/S 750, and support the transfer of data among the hardware devices. The BIOS is stored in some type of read-only-memory, such as ROM, PROM, EPROM, EEPROM or the like, so that the BIOS can be executed when the computer 700 is activated.

When the computer 700 is in operation, the processor 710 is configured to execute software stored within the memory 720, to communicate data to and from the memory 720, and to generally control operations of the computer 700 pursuant to the software. The application 760 and the O/S 750 are read, in whole or in part, by the processor 710, perhaps buffered within the processor 710, and then executed.

When the application 760 is implemented in software it should be noted that the application 760 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 760 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The present invention may be a system, a method, and/or a computer program product. The computer program product may include a computer readable storage medium (or media) having computer readable program instructions thereon for causing a processor to carry out aspects of the present invention.

The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing.

Aspects of the present invention are described herein with reference to flowchart illustrations and/or block diagrams of methods, apparatus (systems), and computer program products according to embodiments of the invention. It will be understood that each block of the flowchart illustrations and/or block diagrams, and combinations of blocks in the flowchart illustrations and/or block diagrams, can be implemented by computer readable program instructions.

A single processor or other unit may fulfill the functions of several items recited in the claims.

It will be understood that the disclosed methods are computer-implemented methods. As such, there is also proposed a concept of a computer program comprising code means for implementing any described method when said program is run on a processing system.

The skilled person would be readily capable of developing a processor for carrying out any herein described method. Thus, each step of a flow chart may represent a different action performed by a processor, and may be performed by a respective module of the processing processor.

As discussed above, the system makes use of a processor to perform the data processing. The processor can be implemented in numerous ways, with software and/or hardware, to perform the various functions required. The processor typically employs one or more microprocessors that may be programmed using software (e.g. microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted that the term "adapted to" is intended to be equivalent to the term "configured to". Any reference signs in the claims should not be construed as limiting the scope.

## Claims

1. A system (300) for generating a model of the airway of a sleeping subject, the system comprising:
an interface (310) adapted to obtain an airway model configured to represent respiratory airflow in the airway of a subject;
a movement sensor arrangement (330) configured to detect movement of the chest or abdomen of a subject;
an airflow sensor arrangement (340) configured to detect an airflow in the airway of the subject; and
a processor arrangement (320) configured to:
determine a movement pattern from detected movement of the chest or abdomen of the subject;
determine an airflow pattern from detected airflow in the airway of the subject;
modify the airway model based on the movement pattern and the airflow pattern so as to generate a subject-specific airway model for the subject representing respiratory airflow in the airway of the subject in a sleep state.

2. The system of claim 1, wherein the airway model comprises a circuit arrangement (400) configured to represent the airway of a subject,
and wherein the circuit arrangement comprises:
one or more passive circuit elements (420), each passive circuit element being configured to represent a respective anatomical structure of the airway; and
one or more active circuit elements (410), each active circuit element being configured to represent a respective driving force for airflow in the airway.

3. The system of claim 2, wherein modifying the airway model comprises:
determining a value of airway tissue compliance based on at least one of the movement pattern and the airflow pattern; and
modifying at least one of the one or more passive circuit elements (420) based on the determined value of airway tissue compliance

4. The system of claim 2 or 3, wherein modifying the airway model comprises:
determining a value of respiration drive based on at least one of the movement pattern and the airflow pattern; and
modifying at least one of the one or more active circuit elements (410) based on the determined value of respiration drive.

5. The system of any of claims 2 to 4, wherein modifying the airway model comprises:
determining a value of airflow based on at least one of the movement pattern and the airflow pattern; and
modifying at least one of the one or more passive circuit elements (420) based on the determined value of airflow.

6. The system of any of claims 2 to 5, wherein the one or more passive circuit elements (420) comprises:
a complex impendence for representing an airway segment of the airway, wherein the real part (422) of the complex impedance is configured to represent a dimension of the airway segment, and wherein the imaginary part (424) of the complex impedance is configured to represent an airflow compliance of the airway segment.

7. The system of any of claims 2 to 6, wherein the one or more active circuit elements (410) comprises:
a current source (412) for representing a muscular-based respiration drive for airflow in the airway.

8. The system of any of claims 2 to 7, wherein the one or more active circuit elements (410) comprises at least one of:
a voltage source (414) for representing a fixed respiration drive for airflow in the airway;
a voltage source for representing a positive airway pressure therapy for airflow in the airway; and
a voltage source for representing a mechanical ventilation therapy for airflow in the airway.

9. The system of any one of claims 1 to 8, further comprising a supplementary sensor (350) arrangement configured to detect a physiological characteristic of the subject,
and wherein the processor arrangement is further adapted to generate the subject-specific airway model for the subject further based on the detected physiological characteristic of the subject.

10. The system of any of claims 1 to 9, wherein the processor is further adapted to:
analyses, with the subject-specific airway model, at least one of detected movement of the chest or abdomen of the subject and/or detected airflow in the airway of the subject, so as to obtain an analysis result; and
generate an output signal based on the analysis result.

11. A sleep therapy system comprising:
a system (300) for generating a model of the airway of a subject according to any preceding claim; and
controller (360) configured to analyses a subject-specific airway model and to generate a control signal based on the analysis result.

12. The system of claim 11, wherein the sleep therapy system further comprises a breathing ventilation system, and wherein the controller is further adapted to control the breathing ventilation system based on the analysis result.

13. A method for generating a model of the airway of a sleeping subject, the method comprising:
obtaining (510) an airway model configured to represent respiratory airflow in the airway of a subject;
detecting (520) movement of the chest or abdomen of a subject;
detecting (530) an airflow in the airway of the subject;
determining (522) a movement pattern from detected movement of the chest or abdomen of the subject;
determining (532) an airflow pattern from detected airflow in the airway of the subj ect;
modifying (540) the airway model based on the movement pattern and the airflow pattern so as to generate (550) a subject-specific airway model for the subject representing respiratory airflow in the airway of the subject in a sleep state.

14. A method for controlling a sleep therapy system, the method comprising:
generating (610)a model of the airway of the subject according to claim 13;
analyzing (620) the subject-specific airway model; and
generating (630) a control signal based on the analysis result.

15. A computer program comprising computer program code which is adapted, when said program is run on a computer, to implement the method of claim 13 or 14.
